Europäisches Patentamt

**European Patent Office**

Office européen des brevets

(11) Veröffentlichungsnummer: **0 229 251**

**A1**

(12) **EUROPÄISCHE PATENTANMELDUNG**

(21) Anmeldenummer: **86115487.0**

(22) Anmeldetag: **07.11.86**

(51) Int. Cl.³: **A 61 F 13/20**

(30) Priorität: **18.12.85 DE 3544711**

(43) Veröffentlichungstag der Anmeldung:
**22.07.87 Patentblatt 87/30**

(84) Benannte Vertragsstaaten:
**AT BE CH FR GB IT LI NL SE**

(71) Anmelder: **Vereinigte Papierwerke AG**
**Schoppershofstrasse 80**
**D-8500 Nürnberg(DE)**

(72) Erfinder: **Männlein, Elisabeth, Dr.**
**Franzosenweg 24**
**D-8520 Erlangen(DE)**

(72) Erfinder: **Reinheimer, Horst, Dr.**
**Imkerweg 21**
**D-8501 Heroldsberg(DE)**

(74) Vertreter: **Pohl, Hans Ludwig**
**Hefnersplatz 3**
**D-8500 Nürnberg 11(DE)**

(54) **Anal-Tampon.**

(57) Es wird ein Anal-Tampon beschrieben, der aus einem Saugkörper aus Textilwatte oder dergl. besteht und der im Innern des Saugkörpers ein Luftkissen enthält. Der Tampon ist von einer flüssigkeitsdurchlässigen Hülle (3) umgeben, die einseitig zu einer Fahne (4) erweitert ist. Die Fahne (4) kann doppelschichtig ausgebildet sein und in das offene Ende (7) kann ein zangenförmiger Applikator (10) eingesetzt sein.

Fig. 2

EP 0 229 251 A1

0229251

Vereinigte Papierwerke      Schoppershofstr. 80
Schickedanz & Co.          8500 Nürnberg

                              12.12.85 - B 74 D
                              HP/1

## Anal-Tampon

Die Erfindung betrifft einen Anal-Tampon mit einem Saugkörper aus Textil-Watte, Zellstoff-Flocken oder dergl.,
einem im Innern des Saugkörpers angeordneten Luftkissen
und einer flüssigkeitsdurchlässigen Hülle, die den Saugkörper umgibt.

Anal-Tampons dieser Art, jedoch ohne im Saugkörper angeordnetes Luftkissen sind grundsätzlich bekannt. Sie sind
beispielsweise in der deutschen Offenlegungsschrift
14 91 151 beschrieben. Sie dienen als Schutz gegen unerwünschte Sekretion bei Anal-inkontinenten Patienten, aber
auch bei der Behandlung von Gefäßschwächen, die im Anal-
Bereich auftreten können, also beispielsweise Hämorrhoiden oder dergl. Die in der deutschen Offenlegungsschrift
14 91 151 beschriebenen Anal-Tampons haben den Nachteil,
daß das Saugkissen bei Befeuchtung klumpig wird und dabei
einen erheblichen Teil seiner Weichheit verliert. Um diesen Nachteil zu beheben, ist andererseits vorgeschlagen
worden, im Innern des Saugkörpers ein Luftkissen anzuordnen, welches beispielsweise die Form eines allseits geschlossenen mit Luft oder einem anderen Gas gefüllten
Folienschlauches oder auch einer spiralig aufgewickelten
Luftpolsterfolie haben kann. Die Anordnung dieses Luftkissens bewirkt, daß der Saugkörper bei Befeuchtung seine
Elastizität und Weichheit beibehält.

Vereinigte Papierwerke
Schickedanz & Co.

Schoppershofstr. 80
8500 Nürnberg

12.12.85 - B 74 D
HP/2

- 2 -

Der vorliegenden Erfindung liegt die Aufgabe zugrunde,
Anal-Tampons der beschriebenen Art so abzuändern, daß ihr
Gebrauch erleichtert wird. Zur Lösung dieser Aufgabe wird
vorgeschlagen, daß die flüssigkeitsdurchlässige Hülle
einseitig zu einer Fahne erweitert ist. Der Tampon erhält
dadurch eine griffige, weiche, nach einer Seite abstehende Lasche, die es erlaubt, ihn nach Gebrauch leicht wieder zu entfernen. Die zur Fahne erweiterte Hülle dient
also dem gleichen Zweck wie die bekannten Rückholfäden
bei Tampons für die Frauenhygiene.

In weiterer Ausgestaltung der Erfindung wird vorgeschlagen, daß die Fahne zu ihrem vom Saugkörper abgewandten
Ende hin verjüngt ist. Die Erweiterung behält dadurch
ihren ursprünglichen Zweck; sie wird jedoch wegen ihrer
beträchtlichen Verkleinerung beim Gebrauch weniger störend.

Grundsätzlich ist es möglich, die zu einer Fahne erweiterte flüssigkeitsdurchlässige Hülle im Bereich der Erweiterung einschichtig auszuführen. Die Hülle muß in diesem Fall an den Stirnseiten des Tampons sowie an der
Längsseite, an der sich die Erweiterung befindet, durch
eine Schweiß- oder Klebenaht verschlossen sein. Andererseits ist es aber auch möglich, und wird hiermit ausdrücklich vorgeschlagen, die Fahne doppelschichtig auszuführen. In diesem Fall kann die Naht entlang der Längs-

Vereinigte Papierwerke
Schickedanz & Co.

Schoppershofstr. 80
8500 Nürnberg

12.12.85 - B 74 D
HP/3

- 3 -

seite des Tampons entfallen und es sind lediglich die
Verschweißungen quer zur Stirnseite und dann weiter bis
zum Ende der Fahne erforderlich. Die doppelschichtige
Ausführung der Fahne bietet den Vorteil, daß sie an dem
vom Saugkörper abgewandten Ende offengelassen werden
kann, und daß es möglich ist, in diese Öffnung einen zangenförmigen Applikator einzuführen. Ein solcher Applikator bietet nun eine erhebliche Gebrauchshilfe und ermöglicht es, den Tampon unter besonders sauberen und hygienischen Bedingungen einzuführen. Nach dem Einführen wird
der Applikator zurückgezogen, so daß das freie Ende der
fahnenförmigen Verlängerung nun in der bereits beschriebenen Weise als Rückholhilfe verbleibt.

Der Applikator selbst kann aus einem U-förmig gebogenen
Streifen eines geeigneten Materials hergestellt sein,
wobei als Material Pappe hinreichender Stärke oder besser
Kunststoff, beispielsweise Acetat- oder Polycarbonat-Folie, vorgeschlagen wird. In das vom Tampon abgewandte
Ende des U-förmig umgebogenen Applikatorstreifens kann
ein Polster eingesetzt werden, welches beispielsweise aus
einem geeigneten Kunststoffschaum, etwa Polyurethanschaum
oder dergl. besteht. Ein derartiges Polster verhindert
es, daß die Applikatorzange am umgebogenen Ende zu sehr
zusammengepreßt und dadurch möglicherweise geknickt wird.
Ein derartiger Knick würde die Sprungelastizität der Zange zerstören.

Vereinigte Papierwerke
Schickedanz & Co.

Schoppershofstr. 80
8500 Nürnberg

12.12.85 - B 74 D
HP/4

- 4 -

Gemäß einer weiteren Ausführungsform wird vorgeschlagen,
daß die Fahne durch Fältelung verkürzt ist und in den
Zangenspalt eines im Querschnitt kelchförmigen Applikators eingeklemmt ist. Durch die Verkürzung ist es möglich, die Gesamteinheit (Tampon nebst Applikator) kleiner
zu machen. Das Einführen der Fahne in den Zangenspalt
erlaubt es, die kelchförmige Öffnung des Applikators, in
der der Tampon ruht, so niedrig zu halten, daß die Projektion der oberen Kelchränder auf den Tampon die Linie
des maximalen Querschnittes nicht trifft. Dadurch ist
sichergestellt, daß sich der Benutzer des Tampons beim
Einführen an den Applikatorrändern nicht stoßen kann.

Die Erfindung wird im folgenden anhand der beigefügten
Zeichnung näher erläutert. Es stellen dar:

Fig. 1 eine vereinfachte perspektivische Ansicht einer
       Ausführungsform eines Anal-Tampons mit verlängerter Hülle;
Fig. 2 eine andere Ausführungsform mit eingesetztem zangenförmigen Applikator;
Fig. 3 eine vereinfachte perspektivische Ausführungsform
       eines Anal-Tampons mit anderem Applikator;
Fig. 4 einen Querschnitt durch Fig. 3.

Vereinigte Papierwerke          Schoppershofstr. 80
Schickedanz & Co.               8500 Nürnberg

                                12.12.85 - B 74 D
                                HP/5

- 5 -

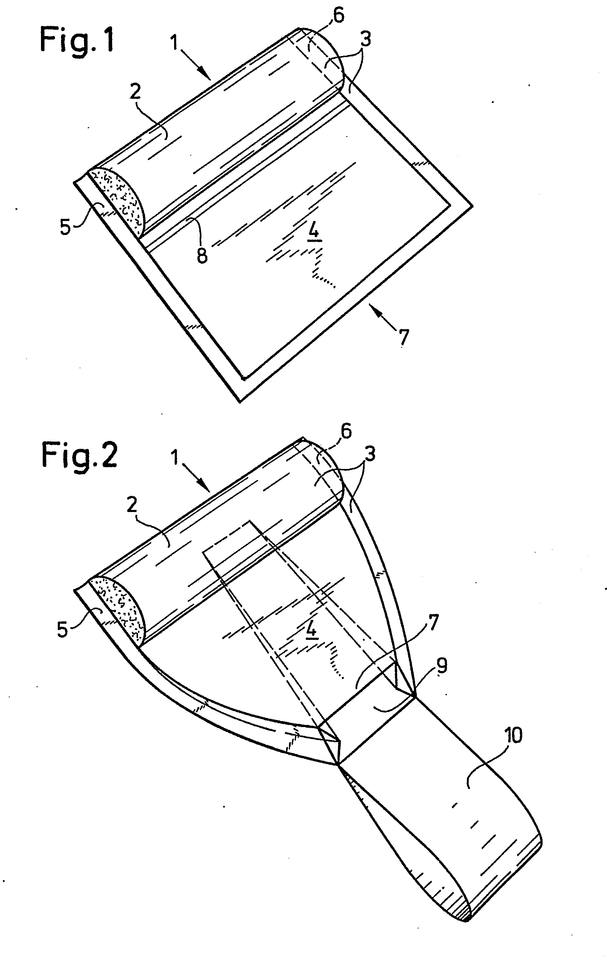

Der in Fig. 1 dargestellte Anal-Tampon ist als Ganzes
mit 1 bezeichnet. Er hat die Form einer langgestreckten
Rolle mit ovalem Querschnitt. Der Tampon besteht aus einem Saugkörper 2, der im dargestellten Ausführungsbeispiel aus einem ungepressten Wickel aus Textil-Watte besteht. Anstelle reiner Textil-Watte kann auch eine Lage
aus Textil-Watte und eine weitere Lage aus Zellstoff-
Flocken verwendet werden, und es können gegebenenfalls
auch die erwähnten Ausgangsstoffe mit Quellstoffen vermischt werden. Um die Fasern des Saugkörpers zusammenzuhalten, ist der Wickel außen mit einer flüssigkeitsdurchlässigen Hülle 3 umgeben, die im Ausführungsbeispiel aus
Vliesstoff besteht. Diese Hülle ist einseitig zu einer
Fahne 4 erweitert, welche als Rückholhilfe beim Gebrauch
des Tampons dient.

Die Fahne 4 kann einschichtig oder doppelschichtig ausgeführt sein. Im Falle der doppelschichtigen Ausführung,
wie in Fig. 1 dargestellt, ist sie an den Stirnseiten des
Saugkörpers durch Schweiß- oder Klebenähte 5; 6 verschlossen, wobei diese Nähte vorteilhafterweise bis zum
freien Ende 7 der Fahne 4 verlängert sind. Zusätzlich
kann noch eine weitere Schweißnaht 8 vorhanden sein, welche dazu dient, den Saugkörper 2 besser in der vorgesehenen Lage zu halten, welche aber nicht unerläßlich ist.

Vereinigte Papierwerke
Schickedanz & Co.

Schoppershofstr. 80
8500 Nürnberg

12.12.85 - B 74 D
HP/6

- 6 -

Andererseits kann die Fahne 4 auch einschichtig ausgeführt sein, in welchem Fall natürlich nicht auf die zusätzliche Schweißnaht 8 verzichtet werden kann, wobei dann aber die Schweißnähte 5 und 6 nur kurz sind und nicht über den Durchmesser des Saugkörpers hinausreichen.

Bei der in Fig. 2 dargestellten Ausführungsform ist die Fahne 4 zu ihrem vom Saugkörper abgewandten Ende 7 hin verjüngt. Die Fahne ist doppelschichtig ausgeführt, so daß am Ende 7 eine Öffnung 9 entsteht, in welche ein zangenförmiger Applikator 10 eingeführt ist. Der Applikator ist dabei so weit in das Innere der Hülle 3 gesteckt, daß er mit seinen vorderen Enden den Saugkörper 2 umfaßt. Es entsteht auf diese Weise eine stabile kompakte Einheit, welche leicht zu handhaben ist. Sobald bei Gebrauch der Tampon eingeführt und an die gewünschte Stelle gesetzt ist, kann der Applikator leicht herausgezogen und gegebenenfalls ein weiteres Mal verwendet werden.

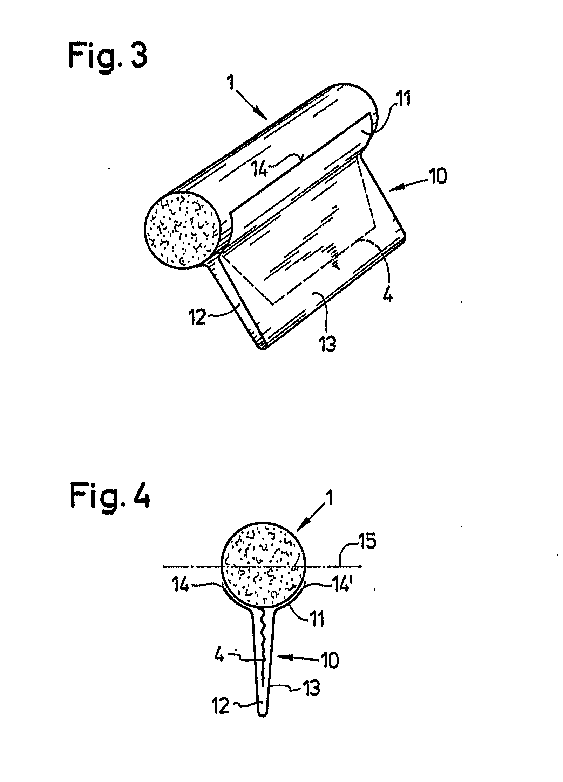

In Fig. 3 ist eine andere Ausführungsform eines Anal-Tampons 1 nebst Applikator 10 dargestellt. Bei dieser Ausführungsform ist der Applikator nicht in das Innere einer Hüllenfahne 4 eingeführt, sondern er umgreift den Tampon an seiner äußeren Oberfläche. Der Applikator weist zu diesem Zweck an seinem dem Tampon zugewandten Ende eine Erweiterung 11 auf, welche im Querschnitt etwa kelchförmig geformt ist. In dieser Erweiterung, die eine Art Trageplattform für den Tampon bildet, ruht der Tamponkörper

Vereinigte Papierwerke
Schickedanz & Co.

Schoppershofstr. 80
8500 Nürnberg

12.12.85 - B 74 D
HP/7

- 7 -

nebst Hülle. Auch in diesem Fall ist die Hülle zu einer
Fahne 4 verlängert, die in Fig. 3 gestrichelt dargestellt
ist. Die Fahne ist in den Spalt 12 des Applikatorgriffs
13 eingeführt und kann dort beim Gebrauch durch Zusammendrücken des Spaltes 12 festgeklemmt werden. Auf diese
Weise ist sichergestellt, daß beim Einführen des Tampons
dieser im Applikator haften bleibt. Ist der Einführvorgang beendet, so wird der Druck auf den Griff 13 nachgelassen und der Applikator kann herausgezogen werden.

In Fig. 4 ist ein Querschnitt durch die Ausführungsform
gemäß Fig. 3 dargestellt. Aus dieser Darstellung ist ersichtlich, daß die Fahne 4 durch Fältelung verkürzt und
in den Spalt 12 des zangenförmigen Applikators 10 eingeführt ist. Desweiteren ist aus Fig. 4 zu ersehen, daß die
Projektion der oberen Applikatorränder 14 und 14' auf die
waagerechte Linie 15, die durch den größten Durchmesser
des Tampons gezogen ist, diese Linie nicht trifft. Dies
bedeutet, daß die Ränder 14; 14' gegenüber dem maximalen
Durchmesser des Tampons etwas zurücktreten, so daß Verletzungen beim Einführen des Tampons nicht eintreten können.

Vereinigte Papierwerke
Schickedanz & Co.

Schoppershofstr. 80
8500 Nürnberg

12.12.85 - B 74 D
HP/9

0229251

-8-

## Bezugszeichenliste

| | | |
|---|---|---|
| 1 | = | Anal-Tampon |
| 2 | = | Saugkörper |
| 3 | = | Hülle |
| 4 | = | Fahne |
| 5 | = | Schweissnaht |
| 6 | = | Schweissnaht |
| 7 | = | freies Ende |
| 8 | = | Schweissnaht |
| 9 | = | Öffnung |
| 10 | = | Applikator |
| 11 | = | Erweiterung |
| 12 | = | Spalt |
| 13 | = | Applikator-Griff |
| 14; 14' | = | obere Applikator-Ränder |
| 15 | = | Linie |

Vereinigte Papierwerke
Schickedanz & Co.

Schoppershofstr. 80
8500 Nürnberg

12.12.85 - B 74 D
HP/8

## Patentansprüche

1. Anal-Tampon mit einem Saugkörper aus Textil-Watte,
   Zellstoff-Flocken oder dergl., einem im Innern des
   Saugkörpers angeordneten Luftkissen und wenigstens
   einer flüssigkeitsdurchlässigen Hülle, die den Saugkörper umgibt,
   dadurch gekennzeichnet,
   daß die flüssigkeitsdurchlässige Hülle (3) einseitig
   zu einer Fahne (4) erweitert ist.

2. Anal-Tampon nach Anspruch 1,
   dadurch gekennzeichnet,
   daß die Fahne (4) zu ihrem vom Saugkörper (2) abgewandten Ende hin verjüngt ist.

3. Anal-Tampon nach Anspruch 1 oder 2,
   dadurch gekennzeichnet,
   daß die Fahne (4) doppelschichtig ist.

4. Anal-Tampon nach Anspruch 3,
   dadurch gekennzeichnet,
   daß das Ende (7) der Fahne (4) offen ist und in die
   Öffnung (9) ein zangenförmiger Applikator (10) eingesetzt ist.

5. Anal-Tampon nach Anspruch 1,
   dadurch gekennzeichnet,
   daß die Fahne (4) durch Fältelung verkürzt ist und in
   den Spalt (12) eines im Querschnitt zangenförmigen und
   zum Tampon hin kelchförmig erweiteren Applikators eingeklemmt ist.

0229251

Fig.1

Fig.2

B 74 D

**Fig. 3**

**Fig. 4**

Europäisches
Patentamt

**EUROPÄISCHER RECHERCHENBERICHT**

| EINSCHLÄGIGE DOKUMENTE | | EP 86115487.0 |
|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | DE - A - 1 491 151 (FOLBERTH)<br>---- | | A 61 F 13/20 |

**RECHERCHIERTE SACHGEBIETE (Int. Cl 4)**

A 61 F

Der vorliegende Recherchenbericht wurde für alle Patentansprüche erstellt.

| Recherchenort | Abschlußdatum der Recherche | Prüfer |
|---|---|---|
| WIEN | 23-03-1987 | FARNIOK |